# FASCICULE DE BREVET EUROPEEN

(11) **EP 3 817 702 B1**
(45) Date de publication et mention de la délivrance du brevet: **20.04.2022**
(21) Numéro de dépôt: 19734120.9
(22) Date de dépôt: 03.07.2019
(51) Int. Cl.: A61H 1/00, A61H 99/00, A47C 1/024, A47C 1/034, A61M 21/02, A61M 21/00, A47B 83/02

(54) **FAUTEUIL DE RELAXATION MULTISENSORIEL**
MEHRSENSORSESSEL
MULTI-SENSORY RECLINER CHAIR

(30) Priorité: 04.07.2018 FR 1856165
(43) Date de publication de la demande: 12.05.2021
(73) Titulaire: Habiche, Leila Benedicte, 98000 Monaco (MC)
(72) Inventeur: Habiche, Leila Benedicte, 98000 Monaco (MC)
(74) Mandataire: Hautier, Nicolas
(86) Numéro de dépôt international: PCT/EP2019/067910
(87) Numéro de publication internationale: WO 2020/007944

(56) Documents cités:
- WO-A1-2012/076248
- WO-A2-2009/068994
- US-A1- 2013 088 059

## Description

### DOMAINE DE L'INVENTION

La présente invention est relative notamment à un fauteuil de relaxation.

Une application préférée concerne l'industrie des appareils multisensoriels permettant la relaxation.

### ARRIERE-PLAN TECHNOLOGIQUE

Dans le domaine des fauteuils de relaxation, on connait des fauteuils 1 qui permettent une inclinaison de l'assise, du repose-jambes et du dossier. L'inclinaison de ces parties du fauteuil permet notamment à l'utilisateur de trouver une position de relaxation améliorée.

Dans le but d'augmenter l'effet de relaxation, certains fauteuils comprennent des stimuli multisensoriels.

C'est notamment le cas de la publication WO2012/076248 qui décrit un fauteuil de relaxation multisensoriel qui combine des stimuli tactiles par le biais de vibrations notamment, olfactifs grâce à des fragrances, visuels par l'intermédiaire d'un écran et auditifs par l'émission de sons.

Ces fauteuils de relaxation permettent ainsi d'agir sur différentes parties du cerveau humain afin de relaxer l'utilisateur.

Ainsi, les fauteuils de relaxation multiplient les sens stimulés afin d'accroitre l'effet de relaxation.

Il existe un besoin constant consistant à améliorer la relaxation de l'utilisateur, ceci sans pour autant rallonger significativement la durée de l'utilisation du fauteuil.

### RESUME DE L'INVENTION

Un aspect de l'invention concerne en particulier un fauteuil de relaxation, de préférence mais non limitativement multisensoriel, comprenant au moins :
- un châssis ;
- une partie supérieure comprenant au moins : un dossier, une assise et un repose-jambes, configurés pour recevoir respectivement le dos, le fessier et les jambes d'un utilisateur, la partie supérieure est supportée par le châssis en étant mobile en rotation par rapport au châssis au moins autour d'un axe vertical,
- un appareil configuré pour générer auprès de l'utilisateur au moins un stimulus, ledit stimulus étant pris parmi l'un des stimuli suivants : sonore, olfactif, visuel, tactile.

Avantageusement, le fauteuil comprend au moins un dispositif de calibrage d'une orientation de référence autour de l'axe vertical et relativement à la direction du champ magnétique terrestre et de préférence par rapport au nord magnétique terrestre;
- le fauteuil comprend au moins un module de contrôle configuré pour recevoir au moins une donnée personnelle d'un utilisateur et pour calculer, à partir de l'au moins une donnée personnelle et de la position de référence du fauteuil, une orientation calculée du fauteuil selon une orientation cardinale autour de l'axe vertical,
- le fauteuil comprend un système de rotation configuré pour entrainer en rotation la partie supérieure autour de l'axe vertical et l'amener jusqu'à l'orientation calculée.

Cette disposition avantageuse permet d'orienter la position du fauteuil, plus particulièrement la position de la partie supérieure du fauteuil, en fonction de la personne et donc d'améliorer l'effet de la relaxation. Dans le cadre du développement de la présente invention il est apparu qu'une relaxation orientée dans l'espace et en fonction de l'utilisateur permet d'améliorer l'efficacité des stimuli et ainsi d'améliorer l'état de relaxation de l'utilisateur. Le fauteuil comprend aussi des moyens pour calculer automatiquement la position de relaxation optimale pour l'utilisateur ainsi que les moyens pour entrainer la rotation du fauteuil. Ces éléments permettent d'automatiser le processus. Ce qui entraine un effort minimal de l'utilisateur pour la préparation de sa séance de relaxation. Cela contribue donc à encore améliorer l'efficacité d'une séance de relaxation.

En partant des dispositifs connus, une fois ces stimuli activés, il est très difficile d'accroitre encore la relaxation de l'utilisateur. En effet, l'augmentation de l'intensité des stimuli n'a pas pour effet l'augmentation de la relaxation, bien au contraire, une intensité trop importante des stimuli peut entraîner un état de stress chez l'utilisateur.

L'invention permet d'accroitre directement l'efficacité de la relaxation et indirectement la réception des différents stimuli par l'utilisateur.

L'invention concerne aussi un système comprenant un fauteuil selon l'invention, comprenant une interface utilisateur configurée pour recevoir l'au moins une donnée personnelle.

L'interface utilisateur permet une meilleure gestion des programmes et des données de l'utilisateur. De plus, la création d'une interface utilisateur permet aussi de faire varier les données personnelles de l'utilisateur. Ainsi, le système permet au fauteuil de s'adapter à plusieurs types d'utilisateurs. Sans cette interface, le fauteuil serait cantonné à une seule configuration, et donc à un seul utilisateur.

### BREVE INTRODUCTION DES FIGURES

D'autres caractéristiques, buts et avantages de la présente invention apparaitront à la lecture de la description détaillée qui suit, et en regard des dessins annexés donnés à titre d'exemples non limitatifs et sur lesquels :
- la figure 1 montre une vue schématique de profil d'un exemple de fauteuil selon l'invention ;
- la figure 2 est une vue de dessus d'un exemple de système de rotation incluant un disque optique avec un code de Grey ;
- la figure 3 est une représentation des secteurs de rotation possibles ;
- la figure 4 est un exemple de disque optique utilisant un code de Grey.

### DESCRIPTION DETAILLEE

Avant d'entrer dans le détail de formes préférées de réalisation de l'invention, en référence aux dessins notamment, d'autres caractéristiques optionnelles de l'invention, qui peuvent être mises en oeuvre de façon combinée selon toutes combinaisons ou de manière alternative, sont indiquées ci-après :
- selon un exemple de réalisation, au moins l'un et de préférence chacun des assise, dossier et repose jambes peuvent s'incliner relativement à un plan horizontal;
- le dispositif de calibrage est configuré de sorte à recevoir la direction du champ magnétique terrestre de la part d'un instrument configuré pour déterminer la direction du champ magnétique terrestre et fournir la direction du champ magnétique terrestre audit module de contrôle, ledit instrument étant pris parmi une boussole, un compas sec, un compas liquide, compas gyroscopique, un magnétomètre;
- l'instrument configuré pour déterminer la direction du champ magnétique terrestre est porté par, rapporté sur, ou logée à l'intérieur du fauteuil;
- ledit instrument est déporté à distance du module de contrôle et du système de rotation;
   Ainsi, le module de contrôle et le système de rotation ne perturbent pas magnétiquement ledit instrument;
- ledit instrument est verticalement positionné au-dessus de l'utilisateur lorsque l'utilisateur est assis dans le fauteuil;
   Ainsi, le module de contrôle et le système de rotation ne perturbent pas magnétiquement ledit instrument;
   Par exemple ledit instrument est positionné au-dessus et au droit de l'assise ou du repose-jambes;
- ledit instrument est enveloppé, au moins partiellement, d'un blindage magnétique, fait en partie au moins par exemple de mu métal;
- l'instrument configuré pour déterminer la direction du champ magnétique terrestre est déporté relativement au fauteuil et dans lequel le dispositif de calibrage comprend une interface de communication filaire ou sans fil avec ledit instrument pour recevoir la direction du champ magnétique terrestre déterminée par ledit instrument;
- le dispositif de calibrage comprend une interface utilisateur configurée pour permettre à un opérateur ou un utilisateur du fauteuil d'entrer des informations relatives à la direction du champ magnétique terrestre;
   Ainsi, dans ces deux précédents exemples de réalisation, ledit instrument n'est pas intégré dans le fauteuil; Il n'est pas porté par le fauteuil; Il est disposé à distance du fauteuil; Selon le premier exemple, le fauteuil comprend une interface de communication avec l'instrument par exemple communication filaire ou une communication sans fil; Alternativement, le fauteuil comprend, par exemple porte, une interface utilisateur par laquelle un opérateur ou utilisateur du fauteuil peut entrer des informations, telle que la direction du champ magnétique terrestre déterminée par l'instrument ;
   Ces deux modes de réalisation peuvent être présents sur un même fauteuil; Ainsi, il peut y avoir plusieurs façons de calibrer l'orientation d'un même fauteuil;
- le module de contrôle comprend une mémoire, un microprocesseur et une mémoire non volatile configurés pour calculer une orientation de la partie supérieure du fauteuil et sauvegarder dans un journal d'événements l'ensemble des positions et rotations de la partie supérieure du fauteuil;
- le sens de rotation de la partie supérieure du fauteuil est déterminé en fonction des précédents sens de rotation de la partie supérieure du fauteuil et de préférence de manière à ce que le fauteuil n'effectue pas plus de deux tours et de préférence un tour par rapport à une orientation initiale;
   Cela permet d'éviter qu'un câble dont une extrémité est solidaire de la partie supérieure soit étiré;
- le système de rotation comprend une denture circulaire interne solidaire de l'assise au moins lors de la rotation du fauteuil autour de l'axe vertical, un pignon porté par un arbre de sortie d'un moteur solidaire du châssis, le pignon étant configuré pour engrainer avec la denture circulaire interne de sorte à entraîner en rotation la denture circulaire interne et l'assise autour de l'axe vertical lorsque le moteur est activé;
- le châssis est configuré pour être solidaire du sol lors de la rotation de la partie supérieure du fauteuil;
- l'axe de rotation du pignon est désaxé relativement à un centre de la denture circulaire interne;
- la denture circulaire interne est logée dans l'assise ou est logée au niveau de l'assise;
- le module de contrôle et le système de rotation du fauteuil sont logés dans un logement situé entre l'assise et le sol sur lequel repose le châssis, le logement formant par exemple un cylindre;
- le logement est recouvert d'un matériau insonorisant;
- l'interface utilisateur est intégrée dans le fauteuil;
- un appareil est configuré pour permettre à l'utilisateur de saisir des données personnelles et pour transmettre lesdites données personnelles au module de contrôle, ledit appareil étant un téléphone intelligent, une tablette tactile ou une montre connectée et dans lequel l'interface utilisateur est déportée du fauteuil et est comprise dans ledit appareil;

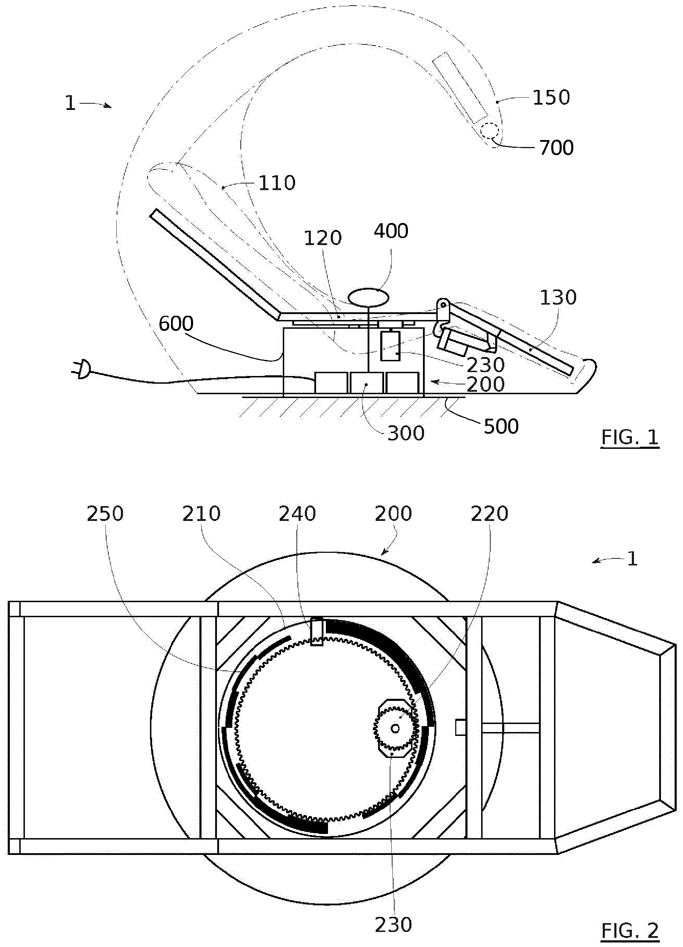

En se référant aux figures 1 à 4, l'invention est relative à un système de relaxation multisensoriel comprenant notamment un fauteuil 1. Ledit fauteuil 1 comprenant de préférence un châssis 500 reposant au sol et faisant office de pied pour une partie supérieure du fauteuil. La partie supérieure comprend au moins un dossier 110 et une assise 120. De préférence la partie supérieure comprend également un repose-jambes 130. Le dossier 110, l'assise 120 et le repose-jambes 130 sont configurés pour recevoir respectivement le dos, le fessier et les jambes d'un utilisateur. Afin d'améliorer la position de l'utilisateur et augmenter l'effet de relaxation dudit utilisateur, chacun des assises 120, dossier 110 et repose-jambes 130 peut s'incliner relativement à un plan horizontal. De préférence, le dossier 110 est incliné de 135° relativement à l'horizontal. De préférence le fauteuil 1 comprend des moteurs 230, de préférence électriques, pour chacune des articulations entre le dossier 110 et l'assise 120 et entre l'assise 120 et le repose-jambes 130.

Les inclinaisons par rapport à l'horizontale du dossier 110, de l'assise 120 et du repose-jambes 130 permettent notamment de positionner l'utilisateur dans une position ergonomique et confortable pour l'utilisateur. Avantageusement l'assise 120 comprend un matelas ayant une épaisseur comprise entre 4 et 20 centimètres (cm) et de préférence entre 8 et 15 cm et de préférence de 11 cm. Avantageusement le matelas est fabriqué en fibres naturelles. De plus, les tibias des jambes peuvent être relevées en ligne avec les cuisses.

Le fauteuil 1 comprend aussi des repose-bras 140 également désignés accoudoirs. Le fauteuil 1 comprend une extension 150 s'étendant au-dessus du dossier 110, voire au-dessus de l'assise 120. L'extension 150 peut également se prolonger de manière à être disposé verticalement au droit du repose jambe 130 ou d'une partie du repose jambe 130. L'extension 150 depuis une extrémité distale du dossier 110. L'extension 150 a de préférence une forme courbée dont l'extrémité distale tend à se rapprocher du repose-jambes 130. Avantageusement, l'extrémité distale le l'extension 150 est face à la tête de l'utilisateur lorsque ce dernier est assis dans le fauteuil 1. Ainsi, de préférence, l'extension 150 est solidaire de la partie supérieure du fauteuil 1.

Le système comprend avantageusement un appareil configuré pour générer auprès de l'utilisateur au moins un stimulus, ledit stimulus étant pris parmi l'un des stimuli suivants : sonore, olfactif, visuel, tactile. Avantageusement l'appareil est configuré pour générer auprès de l'utilisateur au moins un stimulus inclus dans le fauteuil 1.

De préférence, le stimulus visuel consiste en l'émission d'images, ou de vidéo, et/ou de couleurs. Ainsi, le fauteuil 1 est équipé d'un écran et de bandes à diodes électroluminescentes (LED). Avantageusement, l'écran est positionné à l'extrémité distale de l'extension 150 en regard de du dossier 110 du fauteuil 1.

Avantageusement, le fauteuil 1 comprend au moins deux bandes de LED blanches, dont la température de couleur est comprise entre 5000 et 7000 Kelvin et de préférence entre 6000 Kelvin et 6800 Kelvin et de préférence de 6500 Kelvin. De préférence chacune des bandes LED à une dimension en longueur comprise entre 2 et 3 mètres (m) et de préférence entre 2,20 m et 2,60 m et de préférence 2,40 m.

Avantageusement, les bandes LED comprennent un modulateur d'intensité afin d'adapter le stimulus à l'utilisateur.

Le fauteuil 1 comprend aussi des moyens pour réaliser de la chromothérapie. Ainsi, le fauteuil 1 comprend entre sept et vingt et une bandes de LED monochromatiques, et de préférence quatorze bandes de LED monochromatiques.

Avantageusement les bandes de LED monochromatiques émettent au moins de la lumière de couleur rouge, orange, jaune, vert, bleu, indigo, violet. De préférence, la longueur totale des bandes de LED monochromatiques est comprise entre 9 m et 20 m et de préférence entre 14 m et 18 m, et de préférence de 16,8 mètres.

Avantageusement, les bandes LED blanches et monochromatiques sont positionnées dans l'extension 150 du fauteuil 1 en vis-à-vis du dossier 110 et de l'assise 120.

Le fauteuil 1 peut comprendre aussi une paire de lunettes LED permettant de réaliser une autre stimulation visuelle.

Le fauteuil peut comprendre un système de stimulation tactile. Ainsi, des vibrations peuvent, par exemple, être réalisées par le rotor d'un moteur 230 électrique ou par un générateur de fonctions doté d'un amplificateur basse fréquence et d'un caisson de graves permettant la reproduction de vibrations subsoniques et de basses fréquences. Le contrôle de ce module se fait séparément du son du système sur un canal dédié. Avantageusement, le dossier 110, l'assise 120 et le repose-jambes 130 peuvent être chacun équipés d'un appareil de stimulation tactile.

L'appareil de stimulation tactile présent dans le dossier 110, l'assise 120 et le repose-jambes 130 peut être par exemple un caisson de grave permettant la reproduction de vibrations subsoniques et de basses fréquences ou encore d'un système de massage, tel que des bras articulés à l'intérieur du dossier 110, de l'assise 120 et/ou du repose-jambes 130.)

Avantageusement, le fauteuil comprend un générateur de fonctions doté d'un amplificateur basse fréquence et d'un haut-parleur de caisson de basse permettant la reproduction de vibrations subsoniques et basses fréquences capable d'atteindre des fréquences plus basses même que la résonance de Schumann. Le contrôle de ce module se fait séparément du son du système sur un canal dédié mais supporte également les programmes qui le nécessitent.

Le stimulus sonore est avantageusement réalisé par l'incorporation dans le fauteuil 1 d'au moins un haut-parleur. Avantageusement, les haut-parleurs sont positionnés près du dossier 110 et à proximité de la tête de l'utilisateur.

Alternativement, les haut-parleurs peuvent prendre la forme d'un casque audio (intra ou extra-auriculaire) ce qui permet une stimulation sonore plus efficace. Dans une réalisation de l'invention, le fauteuil 1 comprend des haut-parleurs et une capacité à brancher un casque audio. Ainsi, les haut-parleurs effectuent la stimulation sonore en cas d'absence de branchement d'un casque audio.

La stimulation olfactive est, quant à elle, réalisée par une boite d'arômes dans laquelle sont stockés des arômes. Lesdits arômes étant délivrés lors d'une séance de relaxation. Avantageusement, la boite d'arômes est positionnée derrière le dossier 110. Le fauteuil 1 peut aussi comprendre un système d'ionisation situé sur l'extension 150 et en vis-à-vis du dossier 110.

Afin d'augmenter l'efficacité de l'ensemble de ces stimuli, le fauteuil 1 prend aussi en considération son orientation dans l'espace.

En effet, il apparait que la position de l'utilisateur relativement à un champ magnétique, et plus précisément le champ magnétique terrestre, modifie l'efficacité d'une séance de relaxation.

Pour ce faire, le fauteuil 1 comprend un système de rotation 200 et un module de contrôle 300 afin d'orienter la partie supérieure fauteuil 1 autour d'un axe vertical. L'extension 150, lorsqu'elle est présente est donc également entraînée en rotation par rapport au châssis 500.

La détermination de l'orientation du champ magnétique terrestre se fait avantageusement par un instrument 700 configuré pour détecter ladite orientation du champ magnétique terrestre.

Cette détection peut notamment se faire à l'aide d'une boussole, d'un compas sec, un d'un compas liquide, d'un compas gyroscopique ou encore par un magnétomètre.

Dans une réalisation de l'invention, le fauteuil 1 est équipé directement de l'instrument 700 de détection de l'orientation du champ magnétique terrestre.

Dans une autre réalisation de l'invention, le fauteuil 1 est calibré dans une position de référence 0 à l'aide d'un instrument 700 de détection de l'orientation du champ magnétique terrestre. L'instrument 700 de détection de l'orientation du champ magnétique terrestre peut alors ne pas être présent sur le fauteuil 1. Par exemple, l'instrument 700 est embarqué dans un appareil mobile tel qu'un téléphone intelligent, une tablette graphique ou une boussole portable. Dans cette réalisation, la position de référence 0 est de préférence une position dans laquelle l'utilisateur fait face au pôle nord, et de préférence au pôle nord magnétique.

Dans la réalisation dans laquelle l'instrument 700 de détection de l'orientation du champ magnétique terrestre est présent sur le fauteuil 1, ce dernier est avantageusement positionné sur l'extension 150 du fauteuil 1. Le positionnement de l'instrument 700 de détection de l'orientation du champ magnétique terrestre sur ou dans l'extension 150 permet de limiter les risques d'interférences avec d'autres éléments du fauteuil 1 pouvant perturber la détection du champ magnétique terrestre. C'est notamment le cas à proximité des moteurs 230 électriques ou des haut-parleurs qui équipent le fauteuil 1.

Avantageusement, un blindage magnétique entoure au moins partiellement l'instrument 700 de détection de l'orientation du champ magnétique terrestre afin de l'isoler des interférences créées par les autres éléments du fauteuil 1. Avantageusement le blindage est réalisé en Mu-Métal. Le Mu-Métal est un alliage de nickel et de fer. Le blindage peut par exemple prendre la forme d'un ruban adhésif ou d'une plaque autocollante.

Avantageusement, le module de contrôle 300 permet notamment de déterminer le sens et la durée de la rotation du fauteuil 1. Pour ce faire, le module de contrôle 300 comprend avantageusement un microprocesseur, une mémoire, une mémoire non volatile, un programme de gestion du fauteuil 1, et des moyens de pilotage du système de rotation 200 du fauteuil 1. Le module de contrôle 300 comprend aussi un journal des évènements qui recense notamment l'ensemble des mouvements réalisés par le fauteuil 1. Le module de contrôle 300 permet aussi de contrôler l'ensemble des autres stimuli du fauteuil 1. Pour arriver à ce résultat, le module de contrôle 300 stocke, dans sa mémoire non volatile par exemple, des programmes de relaxation. Un pilotage du fauteuil 1 est ensuite réalisé afin de restituer ledit programme de relaxation préalablement enregistré.

Le module de contrôle 300 est aussi configuré pour recevoir au moins une information personnelle de l'utilisateur et la position de référence 0 du fauteuil 1.

L'information personnelle de l'utilisateur peut être par exemple sa date de naissance, son lieu de naissance, son genre biologique (sexe féminin ou masculin).

Le module de contrôle 300 calcule, à partir de données personnelles de l'utilisateur et de la position de référence du fauteuil 1, une orientation du fauteuil 1 selon une orientation cardinale. Comme nous l'avons mentionné précédemment, une position de référence 0 est déterminée en fonction du pôle nord et de préférence du pôle nord magnétique.

La détermination de la position de référence 0 est faite notamment lors de l'installation du fauteuil 1. Ainsi, la partie supérieure du fauteuil 1 est placée dans la position souhaitée grâce au menu de maintenance du programme de gestion, une page d'étalonnage d'orientation est accessible. La rotation de la chaise est ordonnée au système de rotation 200 à travers les options « droite », « gauche », « confirmé » jusqu'à ce que la chaise soit amenée dans la position de référence 0. A ce moment la position de référence 0 est confirmée avec l'option « O ». La position de référence 0 est associée au degré 0 permettant d'avoir une référence absolue.

Cette position de référence 0 peut être calculée en temps réel lorsque l'instrument 700 de détection de l'orientation du champ magnétique terrestre est présent sur le fauteuil 1, ou calibrée en amont lorsque ledit instrument 700 de détection de l'orientation du champ magnétique terrestre est n'est pas présent sur le fauteuil 1.

La détermination de la rotation angulaire du fauteuil 1 se fait notamment relativement aux données personnelles de l'utilisateur. Par exemple, le cercle de rotation du fauteuil 1 est divisé en plusieurs secteurs angulaires correspondant à des orientations cardinales. Avantageusement, le cercle de rotation du fauteuil 1 est divisé en au moins quatre secteurs angulaires égaux de 90° et de préférence en huit secteurs angulaires égaux de 45° et de préférence en douze secteurs angulaires égaux de 30° et de préférence en seize secteurs angulaires égaux de 22,5°. Avantageusement, chaque secteur angulaire correspond à des points cardinaux ou à des points intercardinaux, avec comme point de référence 0 le nord et de préférence le nord magnétique. De préférence, les secteurs angulaires sont regroupés en au moins quatre et de préférence huit zones de 45°. La zone comprenant la position de référence 0 est appelée « Nord ». Ensuite, les zones sont appelées suivant un sens anti-trigonométrique : « Nord-Est », « Est », « Sud-Est », « Sud », « Sud-Ouest », « Ouest » et « Nord-Ouest ».

Dans le cadre de l'invention, lorsque seize secteurs angulaires sont déterminés, ils prennent les références suivantes : Position de référence 0 : N, puis dans un sens anti-trigonométrique les références sont classées de préférence comme il suit : NNE, NE, ENE, E, ESE, SE, SSE, S, SSO, SO, OSO, O, ONO, NO et NNO.

Dans cette réalisation la zone :
- « Nord » comprend les secteurs de références N et NNE;
- « Nord-Est » comprend les secteurs de références NE et ENE ;
- « Est » comprend les secteurs de références E et ESE ;
- « Sud-Est » comprend les secteurs de références SE et SSE;
- « Sud » comprend les secteurs de références S et SSO;
- « Sud-Ouest » comprend les secteurs de références SO et OSO;
- « Ouest » comprend les secteurs de références O et ONO;
- « Nord-Ouest » comprend les secteurs de références NO et NNO.

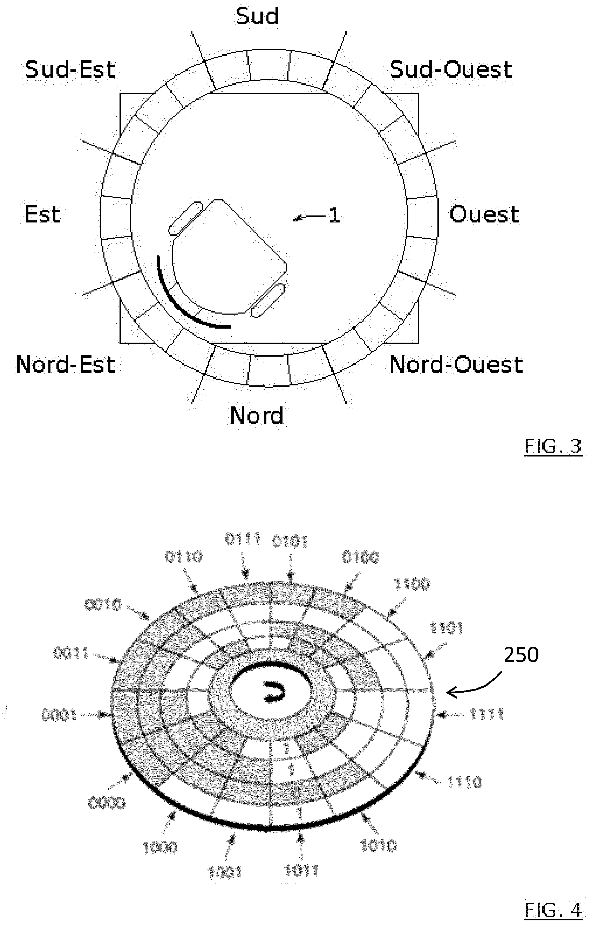

Chaque secteur angulaire est de préférence codé sur quatre bits avec un code de Grey ou code binaire réfléchi. Le code de Grey permet notamment de ne modifier qu'un seul bit à la fois quand un nombre est augmenté d'une unité. L'ensemble des positions est reporté sur un disque optique 250 (un exemple de disque optique 250 est présent en figure 4 avec le code de Grey associé). L'avantage du code de Grey est qu'il est possible et nécessaire de ne faire varier qu'une seule case du disque optique 250 pour passer d'une position à l'autre. Un lecteur optique 240 présent sur le système de rotation 200 permet de lire le disque optique 250. Avantageusement, le disque optique 250 se situe sur la denture circulaire interne 210. Dans un autre mode de réalisation, le disque optique 250 est situé sur le système de rotation 200, ou sur un élément fixe relativement au système de rotation, et le lecteur optique 240 est positionné sur la denture circulaire interne 210. Avantageusement, l'ensemble de ces réalisations permettent de déterminer la position du fauteuil 1 à un instant T.

Le module de contrôle 300 calcule en fonction des données personnelles de l'utilisateur quel est le point cardinal cible ou le point intercardinal cible préférentiel pour une relaxation améliorée. Une fois le point cardinal ou intercardinal déterminé en fonction des données personnelles de l'utilisateur, le module de contrôle 300 détermine la position réelle du fauteuil 1 relative à la position de référence 0. Cette détermination de la position réelle est réalisée grâce aux informations récupérées par le lecteur optique 240 et le disque optique 250. Le module de contrôle 300 détermine ensuite un degré de rotation qui doit être effectué par le fauteuil 1 pour aller au point cardinal cible.

Avantageusement, le module de contrôle 300 calcule deux rotations, à savoir une rotation dans un sens trigonométrique et une rotation dans un sens anti-trigonométrique afin de déterminer quelle est la rotation la plus courte.

Afin de déterminer quel est le sens de rotation que le fauteuil 1 doit réaliser, le module de contrôle 300 prend en compte les différents sens et degrés de rotation réalisés précédemment par le fauteuil 1 depuis la position de référence 0.

Il est précisé qu'un degré maximal de rotation dans un sens est déterminé notamment en fonction de la longueur des différents câbles qui équipent le fauteuil 1.

Typiquement, ces câbles présentent une première extrémité solidaire du châssis 500 ou solidaire du sol et une deuxième extrémité solidaire de la partie supérieure du fauteuil 1. De préférence, le module de contrôle 300 est configuré de sorte à ce que la rotation de la partie supérieure du fauteuil n'entraîne pas une rotation des câbles sur eux-mêmes ou autour du logement 600 sur un secteur angulaire supérieur à 480°, de préférence supérieure à 360° et de préférence supérieure à 180°.

En effet, lorsqu'une rotation est effectuée dans un même sens sur un secteur angulaire trop important, les différents câbles vrillent et risquent de rompre ou d'être endommagés. Pour éviter cela, le module de contrôle 300 limite la rotation à une rotation maximale déterminée. Avantageusement, la rotation maximale correspond à une rotation de 360°, et de préférence à une rotation de 180° relativement à la position de référence 0. Ainsi, la rotation qui doit être effectuée dans un sens, additionnée aux précédentes rotations dans le même sens, moins les rotations effectuées dans le sens opposé, ne peut dépasser l'amplitude maximale de rotation, à savoir 360° et de préférence 180°.

Un exemple à titre illustratif est donné ci-dessous :
Le fauteuil 1 est orienté « Est », c'est à dire qu'au moins une rotation depuis la position de référence 0 (position « Nord ») a déjà eu lieu.
Le journal des évènements indique que :
   - une première rotation depuis la position de référence 0 a eu lieu dans un sens trigonométrique pour une rotation de 90° (position « Ouest »).
      Le total de la rotation du sens trigonométrique depuis la position « Nord » est de 90°. Le total de la rotation du sens anti-trigonométrique depuis la position « Nord » est de -90°.
   - Une deuxième rotation a eu lieu pour passer de la position « Ouest » à la position « Sud ». Ce changement de position a été réalisé par une rotation de 90° dans un sens trigonométrique.
      Le total de la rotation du sens trigonométrique depuis la position « Nord » est de 180°. Le total de l'amplitude de rotation du sens anti-trigonométrique depuis la position « Nord » est de -180°.
      Une troisième rotation a eu lieu pour passer de la position « Ouest » à la position « Est ». Ce changement de position a été réalisé par une rotation trigonométrique de 90°.
      Le total de la rotation du sens trigonométrique depuis la position « Nord » est de 270°. Le total de la rotation du sens anti-trigonométrique depuis la position « Nord » est de -270°.

La prochaine position de la partie supérieure du fauteuil 1 calculée par le module de contrôle 300 est la position « Nord-Ouest ». Le module de contrôle 300 détermine que l'amplitude de la rotation dans un sens trigonométrique est de 135°. Le module de contrôle 300 calcule aussi que l'amplitude de rotation dans un sens anti-trigonométrique est de 225°. Le sens le plus court pour arriver à la position « Nord-Ouest » depuis la position « Est » est le sens trigonométrique. Cependant, en cas de rotation dans un sens trigonométrique, le total de l'amplitude de rotation depuis la position « Nord » de ce sens de rotation sera de 405° ce qui est supérieur aux 360° autorisé par le module de contrôle. Le total de l'amplitude de rotation du sens anti-trigonométrique depuis la position « Nord » sera quant à lui de -45° ce qui est inférieur à 360°. Ainsi le sens de rotation autorisé est le sens de rotation anti-trigonométrique. Au terme de cette quatrième rotation du fauteuil 1, le total de l'amplitude de rotation du sens trigonométrique depuis la position « Nord » est de 45°. Le total de l'amplitude de rotation du sens anti-trigonométrique depuis la position « Nord » est de -45°.

Le système de rotation 200 comprend au moins une denture circulaire interne 210, et un moteur 230 dont l'arbre de sortie est solidaire d'un pignon 220. De préférence le système de rotation 200 est logé ou encapsulé dans un logement 600 également désigné coffre. De préférence, le logement 600 est réalisé par un cylindre creux fermé à l'une de ces extrémités par l'assise 120. L'autre extrémité du cylindre peut-être ouvert ou peut-être fermé par exemple par un couvercle. Avantageusement, le coffre est recouvert d'un matériau insonorisant, afin de ne pas perturber la relaxation de l'utilisateur lors de l'activation du système de rotation 200.

Avantageusement, la denture circulaire interne 210 est fixe relativement à l'assise 120 210 et de préférence solidaire directement ou indirectement de l'assise 120 210.

Le pignon 220 est configuré pour coopérer avec la denture circulaire interne 210. Avantageusement, la rotation du pignon 220 entraine la rotation de la denture circulaire interne 210, et donc entraine la rotation de l'ensemble de la partie supérieure du fauteuil 1 relativement au sol. Il est précisé que la denture circulaire interne 210 est aussi mobile relativement au disque optique 250.

Un moteur 230, de préférence pas-à-pas, est configuré pour imprimer une rotation sur le pignon 220.

Le moteur 230 et le pignon 220 sont de préférence positionnés à l'intérieur de la denture circulaire interne 210.

Avantageusement, le pignon 220 et le moteur 230 sont dans une position désaxée relativement au centre de la denture circulaire interne 210.

Avantageusement, les données personnelles sont transmises au module de contrôle 300 par l'intermédiaire d'une interface utilisateur. L'interface utilisateur 400 permet d'une part de rentrer les informations personnelles de l'utilisateur et d'autre part de sélectionner un programme de relaxation.

L'interface utilisateur 400 est dans une réalisation de l'invention intégrée au fauteuil 1. Dans une autre réalisation de l'invention, l'interface utilisateur 400 est déportée du fauteuil 1. Dans ce mode de réalisation, l'interface d'utilisation 400 peut prendre la forme d'une application sur un téléphone intelligent ou sur une tablette tactile ou sur tout autre appareil distant. Toujours dans cette réalisation, si l'interface utilisateur 400 est déportée du fauteuil 1, elle comprend des moyens de connexion, notamment à distance, avec le module de contrôle 300. Les moyens de connexions peuvent par exemple être une puce de télécommunication et une antenne présentes dans le module de contrôle 300 et dans l'appareil supportant l'interface utilisateur.

L'interface utilisateur 400 peut aussi prendre la forme d'une télécommande comprenant un émetteur-récepteur radio ou infrarouge.

Avantageusement, le fauteuil 1 est muni de capteurs permettant de détecter les paramètres biométriques de l'utilisateur. Dans une autre réalisation de l'invention, les capteurs permettant de détecter les paramètres biométriques de l'utilisateur sont portés par ledit utilisateur. Dans cette réalisation, l'utiliser porte un bracelet embarquant les capteurs biométriques. Les paramètres biométriques détectés sont, par exemple, le rythme cardiaque, la tension artérielle, l'oxygénation, le rythme respiratoire, le volume respiratoire, la température et la conductivité épidermique. Ces informations sont stockées, dans un programme de suivi dans la mémoire non volatile, et accessibles depuis l'interface utilisateur. Ainsi, l'utilisateur peut avoir un suivi de ses séances de relaxation. Par ailleurs, des programmes personnalisés de relaxation peuvent lui être proposés en fonction de ses paramètres biométriques.

L'invention n'est pas limitée aux modes de réalisation précédemment décrits, mais s'étend à tous modes de réalisation couverts par les revendications.

### REFERENCES

- 1.: Fauteuil
- 110.: Dossier
- 120.: Assise
- 130.: Repose-jambes
- 150.: Extension
- 200.: Système de rotation
- 210.: Denture circulaire interne
- 220.: Pignon
- 230.: Moteur
- 240.: Lecteur optique
- 250.: Disque optique
- 300.: Module de contrôle
- 400.: Interface utilisateur
- 500.: Châssis
- 600.: Logement
- 700.: Instrument

## Revendications

1. Fauteuil (1) de relaxation multisensoriel comprenant au moins :
- un châssis (500) ;
- une partie supérieure comprenant au moins : un dossier (110), une assise (120) et un repose-jambes, configurés pour recevoir respectivement le dos, le fessier et les jambes d'un utilisateur, la partie supérieure est supportée par le châssis (500) en étant mobile en rotation par rapport au châssis (500) au moins autour d'un axe vertical,
- un appareil configuré pour générer auprès de l'utilisateur au moins un stimulus, ledit stimulus étant pris parmi l'un des stimuli suivants : sonore, olfactif, visuel, tactile;
**caractérisé en ce que**:
- le fauteuil (1) comprend au moins un dispositif de calibrage d'une orientation de référence autour de l'axe vertical et relativement à la direction du champ magnétique terrestre et de préférence par rapport au nord magnétique terrestre;
- le fauteuil (1) comprend au moins un module de contrôle (300) configuré pour recevoir au moins une donnée personnelle d'un utilisateur et pour calculer, à partir de l'au moins une donnée personnelle et de la position de référence du fauteuil (1), une orientation calculée du fauteuil (1) selon une orientation cardinale autour de l'axe vertical,
- le fauteuil (1) comprend un système de rotation (200) configuré pour entrainer en rotation la partie supérieure autour de l'axe vertical et l'amener jusqu'à l'orientation calculée.

2. Fauteuil (1) selon la revendication précédente, dans lequel le dispositif de calibrage est configuré de sorte à recevoir la direction du champ magnétique terrestre de la part d'un instrument (700) configuré pour déterminer la direction du champ magnétique terrestre et fournir la direction du champ magnétique terrestre audit module de contrôle (300), ledit instrument (700) étant pris parmi une boussole, un compas sec, un compas liquide, compas gyroscopique, un magnétomètre.

3. Fauteuil (1) selon la revendication précédente dans lequel l'instrument (700) configuré pour déterminer la direction du champ magnétique terrestre est porté par, rapporté sur, ou logée à l'intérieur du fauteuil (1).

4. Fauteuil (1) selon la revendication précédente dans lequel ledit instrument (700) est déporté à distance du module de contrôle (300) et du système de rotation (200) et de préférence ledit instrument (700) est verticalement positionné au-dessus de l'utilisateur lorsque l'utilisateur est assis dans le fauteuil (1).

5. Fauteuil (1) selon l'une quelconque des deux revendications précédentes dans lequel ledit instrument (700) est enveloppé, au moins partiellement, d'un blindage magnétique, fait en partie au moins par exemple de mu métal.

6. Fauteuil (1) selon la revendication 2 dans lequel l'instrument (700) configuré pour déterminer la direction du champ magnétique terrestre est déporté relativement au fauteuil (1) et dans lequel le dispositif de calibrage comprend une interface de communication filaire ou sans fil avec ledit instrument (700) pour recevoir la direction du champ magnétique terrestre déterminée par ledit instrument (700).

7. Fauteuil (1) selon l'une quelconque des revendications précédentes dans lequel le dispositif de calibrage comprend une interface utilisateur (400) configurée pour permettre à un opérateur ou un utilisateur du fauteuil d'entrer des informations relatives à la direction du champ magnétique terrestre.

8. Fauteuil (1) selon l'une des revendications précédentes dans lequel le module de contrôle (300) comprend une mémoire, un microprocesseur et une mémoire non volatile configurés pour calculer une orientation de la partie supérieure du fauteuil (1) et sauvegarder dans un journal d'événements l'ensemble des positions et rotations de la partie supérieure du fauteuil (1) et de préférence dans lequel le sens de rotation de la partie supérieure du fauteuil (1) est déterminé en fonction des précédents sens de rotation de la partie supérieure du fauteuil (1) et de préférence de manière à ce que le fauteuil (1) n'effectue pas plus de deux tours et de préférence pas plus de un tour par rapport à une orientation initiale.

9. Fauteuil (1) selon l'une quelconque des revendications précédentes dans lequel le système de rotation (200) comprend une denture circulaire interne (210) solidaire de l'assise (120) au moins lors de la rotation du fauteuil (1) autour de l'axe vertical, un pignon (220) porté par un arbre de sortie d'un moteur (230) solidaire du châssis, le pignon (220) étant configuré pour engrainer avec la denture circulaire interne (210) de sorte à entraîner en rotation la denture circulaire interne (210) et l'assise (120) autour de l'axe vertical lorsque le moteur (230) est activé.

10. Fauteuil (1) selon la revendication précédente dans lequel l'axe de rotation du pignon (220) est désaxé relativement à un centre de la denture circulaire interne (210).

11. Fauteuil (1) selon l'une quelconque des deux revendications précédentes dans lequel la denture circulaire interne (210) est logée dans l'assise (120) ou est logée au niveau de l'assise (120).

12. Fauteuil (1) selon l'une quelconque des revendications précédentes dans lequel le module de contrôle (300) et le système de rotation (200) du fauteuil (1) sont logés dans un logement (600) situé entre l'assise (120) et le sol sur lequel repose le châssis (500), le logement (600) formant par exemple un cylindre.

13. Fauteuil (1) selon la revendication précédente dans lequel le logement (600) est recouvert d'un matériau insonorisant.

14. Système comprenant un fauteuil (1) selon l'une quelconque des revendications précédentes, comprenant une interface utilisateur (400) configurée pour recevoir l'au moins une donnée personnelle et de préférence dans lequel l'interface utilisateur (400) est intégrée dans le fauteuil (1).

15. Système selon la revendication 14 comprenant un appareil configuré pour permettre à l'utilisateur de saisir des données personnelles et pour transmettre lesdites données personnelles au module de contrôle, ledit appareil étant un téléphone intelligent, une tablette tactile ou une montre connectée et dans lequel l'interface utilisateur (400) est déportée du fauteuil (1) et est comprise dans ledit appareil.

## Patentansprüche

1. Multisensor-Entspannungssessel (1), umfassend mindestens:
- ein Gestell (500);
- einen oberen Abschnitt, umfassend mindestens: eine Rücklehne (110), eine Sitzfläche (120) und eine Beinstütze, die dazu konfiguriert sind, den Rücken, das Gesäß bzw. die Beine eines Benutzers aufzunehmen, wobei der obere Abschnitt von dem Gestell (500) gestützt wird, indem er in Bezug auf das Gestell (500) um mindestens eine vertikale Achse drehbar ist,
- eine Vorrichtung, die dazu konfiguriert ist, in der Nähe des Benutzers mindestens einen Stimulus zu erzeugen, wobei der Stimulus aus einem der folgenden Stimuli ausgewählt ist: akustisch, olfaktorisch, optisch, taktil;
**dadurch gekennzeichnet, dass**:
- der Sessel (1) mindestens ein Gerät zur Kalibrierung einer Referenzausrichtung um die vertikale Achse und relativ zur Richtung des Erdmagnetfelds und vorzugsweise in Bezug auf den magnetischen Norden der Erde umfasst;
- der Sessel (1) mindestens ein Steuermodul (300) umfasst, das dazu konfiguriert ist, mindestens einen persönlichen Datensatz eines Benutzers zu empfangen und aus dem mindestens einen persönlichen Datensatz und der Referenzposition des Sessels (1) eine berechnete Ausrichtung des Sessels (1) gemäß einer hauptsächlichen Ausrichtung um die vertikale Achse zu berechnen,
- der Sessel (1) ein Drehsystem (200) umfasst, das dazu konfiguriert ist, den oberen Abschnitt um die vertikale Achse in Drehung zu versetzen und ihn bis zur berechneten Ausrichtung zu führen.

2. Sessel (1) nach dem vorhergehenden Anspruch, wobei das Kalibriergerät derart konfiguriert ist, dass die Richtung des Erdmagnetfelds des Teils eines Instruments (700) empfangen wird, das dazu konfiguriert ist, die Richtung des Erdmagnetfelds zu bestimmen und die Richtung des Erdmagnetfelds an das Steuermodul (300) bereitzustellen, wobei das Instrument (700) aus einer Bussole, einem Trockenkompass, einem Flüssigkeitskompass, einem Kreiselkompass, einem Magnetometer ausgewählt ist.

3. Sessel (1) nach dem vorhergehenden Anspruch, wobei das Instrument (700), das dazu konfiguriert ist, die Richtung des Erdmagnetfelds zu bestimmen, von dem Sessel (1) getragen, auf dem Sessel (1) aufgebracht oder im Inneren des Sessels (1) untergebracht ist.

4. Sessel (1) nach dem vorhergehenden Anspruch, wobei das Instrument (700) in einem Abstand von dem Steuermodul (300) und dem Drehsystem (200) ausgelagert ist und vorzugsweise wobei das Instrument (700) vertikal über dem Benutzer positioniert ist, wenn der Benutzer in dem Sessel (1) sitzt.

5. Sessel (1) nach einem der zwei vorhergehenden Ansprüche, wobei das Instrument (700) zumindest zum Teil von einer magnetischen Abschirmung umgeben ist, die zumindest zum Teil aus Mu-Metall hergestellt ist.

6. Sessel (1) nach Anspruch 2, wobei das Instrument (700), das dazu konfiguriert ist, die Richtung des Erdmagnetfelds zu bestimmen, relativ zu dem Sessel (1) ausgelagert ist und wobei das Kalibriergerät eine drahtgebundene oder drahtlose Kommunikationsschnittstelle mit dem Instrument (700) umfasst, um die Richtung des Erdmagnetfelds, die von dem Instrument (700) bestimmt wird, zu empfangen.

7. Sessel (1) nach einem der vorhergehenden Ansprüche, wobei das Kalibriergerät eine Benutzerschnittstelle (400) umfasst, die dazu konfiguriert ist, einem Bediener oder einem Benutzer des Sessels zu ermöglichen, Informationen in Bezug auf die Richtung des Erdmagnetfelds einzugeben.

8. Sessel (1) nach einem der vorhergehenden Ansprüche, wobei das Steuermodul (300) einen Speicher, einen Mikroprozessor und einen nichtflüchtigen Speicher umfasst, die dazu konfiguriert sind, eine Ausrichtung des oberen Abschnitts des Sessels (1) zu berechnen und die Gesamtheit von Positionen und Drehungen des oberen Abschnitts des Sessels (1) in einem Ereignisprotokoll zu speichern und vorzugsweise wobei die Drehrichtung des oberen Abschnitts des Sessels (1) in Abhängigkeit von vorhergehenden Drehrichtungen des oberen Abschnitts des Sessels (1) und vorzugsweise derart bestimmt wird, dass der Sessel (1) nicht mehr als zwei Umdrehungen und vorzugsweise nicht mehr als eine Umdrehung in Bezug auf eine anfängliche Ausrichtung durchführt.

9. Sessel (1) nach einem der vorhergehenden Ansprüche, wobei das Drehsystem (200) eine interne kreisförmige Verzahnung (210), die mit der Sitzfläche (120) mindestens während der Drehung des Sessels (1) um die vertikale Achse fest verbunden ist, ein Zahnrad (220), das von einer Abtriebswelle eines Motors (230) getragen wird, der fest mit dem Gestell verbunden ist, umfasst, wobei das Zahnrad (220) dazu konfiguriert ist, in die interne kreisförmige Verzahnung (210) derart einzugreifen, dass die interne kreisförmige Verzahnung (210) und die Sitzfläche (120) um die vertikale Achse in Drehung versetzt wird, wenn der Motor (230) aktiviert wird.

10. Sessel (1) nach dem vorhergehenden Anspruch, wobei die Drehachse des Zahnrads (220) relativ zu einer Mitte der internen kreisförmigen Verzahnung (210) verlagert ist.

11. Sessel (1) nach einem der zwei vorhergehenden Ansprüche, wobei die interne kreisförmige Verzahnung (210) in der Sitzfläche (120) untergebracht ist oder auf der Höhe der Sitzfläche (120) untergebracht ist.

12. Sessel (1) nach einem der vorhergehenden Ansprüche, wobei das Steuermodul (300) und das Drehsystem (200) des Sessels (1) in einem Gehäuse (600) untergebracht sind, das sich zwischen der Sitzfläche (120) und dem Boden befindet, auf dem das Gestell (500) ruht, wobei das Gehäuse (600) beispielsweise einen Zylinder bildet.

13. Sessel (1) nach dem vorhergehenden Anspruch, wobei das Gehäuse (600) durch ein schalldämpfendes Material bedeckt ist.

14. System, umfassend einen Sessel (1) nach einem der vorhergehenden Ansprüche, umfassend eine Benutzerschnittstelle (400), die dazu konfiguriert ist, mindestens einen persönlichen Datensatz zu empfangen, und vorzugsweise wobei die Benutzerschnittstelle (400) in den Sessel (1) integriert ist.

15. System nach Anspruch 14, umfassend eine Vorrichtung, die dazu konfiguriert ist, dem Benutzer zu ermöglichen, persönliche Datensätze zu erfassen, und die persönlichen Datensätze an das Steuermodul zu übertragen, wobei die Vorrichtung ein verbundenes Smartphone, ein verbundener Tabletcomputer oder eine verbundene Uhr ist und wobei die Benutzerschnittstelle (400) aus dem Sessel (1) ausgelagert ist und in dem Gerät enthalten ist.

## Claims

1. A multi-sensory recliner chair (1) comprising at least:
- one frame (500);
- one upper portion comprising at least: a backrest (110), a seat (120) and a leg-rest, configured to receive respectively the back, the gluteus and the legs of a user, the upper portion is supported by the frame (500) while being movable in rotation relative to the frame (500) at least about a vertical axis,
- one device configured to generate, from the user, at least one stimulus, said stimulus being taken from one of the following stimuli: sound, olfactory, visual, tactile stimuli;
**characterised in that**:
- the chair (1) comprises at least one device for calibrating a reference orientation about the vertical axis and relative to the direction of the earth's magnetic field and preferably relative to the earth's magnetic north;
- the chair (1) comprises at least one control module (300) configured to receive at least one personal data item from a user and to calculate, from the at least one personal data item and the reference position of the chair (1), a calculated orientation of the chair (1) according to a cardinal orientation about the vertical axis,
- the chair (1) comprises a rotation system (200) configured to drive in rotation the upper portion about the vertical axis and bring it to the calculated orientation.

2. The chair (1) according to the preceding claim, wherein the calibration device is configured so as to receive the direction of the earth's magnetic field from an instrument (700) configured to determine the direction of the earth's magnetic field and provide the direction of the earth's magnetic field to said control module (300), said instrument (700) being taken from a compass, a dry compass, a liquid compass, gyrocompass, a magnetometer.

3. The chair (1) according to the preceding claim, wherein the instrument (700) configured to determine the direction of the earth's magnetic field is carried by, directly mounted on, or housed inside the chair (1).

4. The chair (1) according to the preceding claim, wherein said instrument (700) is remote from the control module (300) and the rotation system (200) and preferably said instrument (700) is positioned vertically above the user when the user is sitting in the chair (1).

5. The chair (1) according to any one of the two preceding claims, wherein said instrument (700) is enveloped, at least partially, by a magnetic shield, made at least partially for example of mu-metal.

6. The chair (1) according to claim 2, wherein the instrument (700) configured to determine the direction of the earth's magnetic field is deported relative to the chair (1) and wherein the calibration device comprises a wired or wireless communication interface with said instrument (700) to receive the direction of the earth's magnetic field determined by said instrument (700).

7. The chair (1) according to any one of the preceding claims, wherein the calibration device comprises a user interface (400) configured to allow an operator or a user of the chair to enter information relating to the direction of the earth's magnetic field.

8. The chair (1) according to one of the preceding claims, wherein the control module (300) comprises a memory, a microprocessor and a non-volatile memory configured to calculate an orientation of the upper portion of the chair (1) and save, in an event log, all positions and rotations of the upper portion of the chair (1) and preferably wherein the direction of rotation of the upper portion of the chair (1) is determined depending on the previous directions of rotation of the upper portion of the chair (1) and preferably so that the chair (1) does not perform more than two turns and preferably not more than one turn relative to an initial orientation.

9. The chair (1) according to any one of the preceding claims, wherein the rotation system (200) comprises an internal circular toothing (210) secured to the seat (120) at least during the rotation of the chair (1) about the vertical axis, a pinion (220) carried by an output shaft of a motor (230) secured to the frame, the pinion (220) being configured to mesh with the internal circular toothing (210) so as to drive in rotation the internal circular toothing (210) and the seat (120) about the vertical axis when the motor (230) is activated.

10. The chair (1) according to the preceding claim, wherein the axis of rotation of the pinion (220) is offset relative to a centre of the internal circular toothing (210).

11. The chair (1) according to any one of the two preceding claims, wherein the internal circular toothing (210) is housed in the seat (120) or is housed at the seat (120).

12. The chair (1) according to any one of the preceding claims, wherein the control module (300) and the rotation system (200) of the chair (1) are housed in a housing (600) located between the seat (120) and the ground on which the frame (500) rests, the housing (600) forming for example a cylinder.

13. The chair (1) according to the preceding claim, wherein the housing (600) is covered with a sound absorbing material.

14. A system comprising a chair (1) according to any one of the preceding claims, comprising a user interface (400) configured to receive the at least one personal data item and preferably wherein the user interface (400) is integrated into the chair (1).

15. The system according to claim 14, comprising an apparatus configured to allow the user to enter personal data and to transmit said personal data to the control module, said apparatus being a smart phone, a touch pad or a connected watch and wherein the user interface (400) is deported from the chair (1) and is comprised in said apparatus.
